# EUROPÄISCHE PATENTANMELDUNG

(11) **EP 1 736 552 A1**
(43) Veröffentlichungstag der Anmeldung: **27.12.2006**
(21) Anmeldenummer: 05013432.9
(22) Anmeldetag: 22.06.2005
(51) Int. Cl.: C12Q 1/68, C07H 21/00

(54) **Nukleolipide und deren Verwendung, sowie Vorrichtungen zur Nukleinsäureanalytik**

(71) Anmelder: Universität Osnabrück, 49074 Osnabrück (DE)
(72) Erfinder: Rosemeyer, Helmut, Dr., 49076 Osnabrück (DE)
(74) Vertreter: Kröncke, Rolf

(57) **Zusammenfassung**

Gegenstand der Erfindung ist ein Verfahren zur Identifizierung einer unbekannten, mit einer Reportergruppe markierten Nukleinsäure (Targetsequenz) durch basenspezifische Hybridisierung an einer Flüssigkeits-Gas (z.B. Wasser-Luft) oder Flüssig-Flüssig (Wasser-Öl) Grenzfläche mit einer passenden Komplementärsequenz, die zu einer Bibliothek von Sequenzen (= Probenoligonukleotide) gehört. Die Probensequenzen sind dadurch charakterisiert, dass sie alle an einem ihrer Termini (5'-Ende oder 3'-Ende, vorzugsweise am 5'-Ende) ein ein-, doppel- oder mehrkettiges Lipid tragen, die eine Spreitung in monomolekularer Schicht an einer Flüssigkeits-Gas oder Flüssig-Flüssig Phasengrenzfläche ermöglicht. Entsprechende Nukleolipide sind ebenfalls Gegenstand der vorliegenden Erfindung.

## Beschreibung

Gegenstand der Erfindung ist ein Verfahren zur lsolierung und/oder Identifizierung einer unbekannten, mit einer Reportergruppe markierten Nukleinsäuresequenz (Targetsequenz) durch basenspezifische Hybridisierung an einer Flüssigkeits-Gas (z.B. Wasser-Luft) oder Flüssig-Flüssig (Wasser-Öl) Grenzfläche mit einer passenden Komplementärsequenz, die zu einer Bibliothek von Sequenzen ( im Folgenden als Probenoligonukleotide, Probensequenzen oder Probennukleinsäuren bezeichnet) gehört. Die Probensequenzen sind dadurch charakterisiert, dass sie alle an einem ihrer Termini (5'-Ende oder 3'-Ende, vorzugsweise am 5'-Ende) einen ein-, doppel- oder mehrkettigen Lipidanteil tragen, die eine Spreitung in monomolekularer Schicht an einer Flüssigkeits-Gas oder Flüssig-Flüssig Phasengrenzfläche ennöglicht.

### Hintergrund der Erfindung

Nukleinsäuren haben als Träger bzw. Überträger der genetischen Information eine zentrale Bedeutung in der belebten Natur. Mit der zunehmenden Kenntnis der grundlegenden molekularbiologischen Mechanismen ist es in den letzten Jahren möglich geworden, die Neukombination von Genen zu betreiben. Diese Technologie eröffnet z.B. neue Möglichkeiten in der medizinischen Diagnostik und Therapie sowie in der Pflanzenzüchtung.

Ein wichtiges Werkzeug zur Erklärung dieser Zusammenhänge und zur Lösung der Probleme war und ist der Nachweis von Nukleinsäuren und Nukleinsäurefragmenten, und zwar sowohl was ihren spezifischen Nachweis betrifft, als auch was ihre Sequenz, also ihre Primärstruktur angeht. Weiterhin ist ein Bestandteil molekularbiologischen Arbeitens die Isolierung von Nukleinsäuresequenzen.

Die spezifische Nachweisbarkeit von Nukleinsäuren beruht auf der Eigenschaft der Moleküle, mit anderen Nukleinsäuren durch Ausbildung von Basenpaaren über Wasserstoffbrückenbindungen in Wechselwirkung zu treten - zu "hybridisieren". Die Analyse von Genen bzw. Genabschnitten erfolgt heute auf DNA-Chips. Solche Chips oder DNA-Microarrays bestehen aus einem festen Träger (z.B. einem Glasobjektträger), auf dem in einem regelmäßigen Muster einzelsträngige DNA-Moleküle bekannter Sequenz aufgebracht sind. Die Herstellung derartiger Nukleinsäurechips erfolgt entweder (i) durch direkte Synthese mittels Masken und eines photolithographischen Verfahrens auf dem festen Träger, oder (ii) es werden vorgefertigte und terminal funktionalisierte Nukleinsäureproben auf aktivierten Oberflächen chemisch fixiert. Die DNA-Analyse auf dem Chip umfasst mehrere Teilschritte: (i) Probenvorbereitung (Extraktion, PCR etc.); (ii) Hybridisierung auf dem Chip; (iii) Stringentes Waschen; (iv) Detektion; (v) Bioinformatische Auswertung.

Beide Verfahren zur Herstellung von DNA-Chips sind mit zahlreichen Problemen behaftet. Bei dem 1. Verfahren findet die Oligonukleotidsynthese direkt auf dem Träger einschließlich umfangreichen Entschützungsreaktionen und Waschschritte statt. Dieses stellt ein sehr aufwendiges Verfahren dar. Beim 2. Verfahren muss beispielsweise die feste Oberfläche - üblicherweise eine Glasplatte - auf komplizierte Weise mit funktionellen Gruppen aktiviert werden. Erst dann ist das Aufbringen von ebenfalls funktionalisierten vorgefertigten Nukleinsäuren bekannter Sequenz möglich. Dieses Aufbringen ("Spotten") ist ebenfalls problembehaftet und bedarf teurer und komplizierter Geräte, der sogenannten "Microarrayer".

Zur Isolierung von Nukleinsäuren wird heutzutage üblicherweise ein Verfahren unter Verwendung von chaotropen Verbindungen in Kombination mit soliden Materialien, wie Silika oder Silikaderivate oder Magnetpartikel eingesetzt. Der Nachteil dieser Verfahren ist ein aufwendiges Isolierungsverfahren mit vielen Wasch- und Zentrifugationsschritten.

Die Isolierung von mRNA über OligodT-Nukleotide erfordert weiterhin eine Vorbehandlung der soliden Materialien, entweder um eine direkte Kopplung oder um eine indirekte Kopplung der OligodT-Moleküle an die soliden Materialien zu ermöglichen.

Eine Aufgabe der vorliegenden Erfindung ist die Bereitstellung eines neuen, erheblich einfacheren und preiswerteren Verfahren zur Analytik von Nukleinsäuren, die aufwendige chemische Kopplungsreaktionen mit den Trägermaterialien vermeidet.

Eine weitere Aufgabe der vorliegenden Erfindung ist die Bereitstellung neuer Verbindungen, die insbesondere in Verfahren zur Analyse von Nukleinsäuren verwendet werden können.

### Zusammenfassung der Erfindung

Die vorliegende Erfindung stellt neue Nukleolipide bereit, die einen lipophilen Anteil und ein Nukleosid bzw. Oligo- oder Polynukleotid umfassen. Diese beiden Komponenten sind über einen Linker (z.B. ein Stickstoffatom), einen Abstandshalter (auch als Spacer bezeichnet) und eine Konnektor (z.B. einen Phosphordiesterrest) miteinander verbunden. Der Konnektor ist dabei eine Phosphordiesterverbindung, die zwischen dem Lipidanteil und dem Nukleotidanteil ausgebildet wird, wenn eine Phosphonat- oder Phosphoramiditgruppe mit der 3' oder 5' OH-Gruppe des Nukleosids, z.B. dem 3' oder 5' Ende eines Oligonukleotids oder Polynukleotids, umgesetzt wird. Weiterhin stellt die vorliegende Erfindung Verbindungen bereit, die als Ausgangsverbindungen für die Herstellung der oben genannten Nukleolipide geeignet sind. Diese Verbindungen, in Folgenden auch als reaktive Lipide bezeichnet, umfassen einen lipophilen Anteil und eine funktionelle Gruppe, die Ober einen Linker und einen Abstandshalter miteinander verbunden sind, dadurch gekennzeichnet, dass der Linker eine Amingruppe oder ein Molekül mit mindestens zwei funktionellen Gruppen ist und mit dem lipophilen Anteil direkt verbunden ist und die funktionelle Gruppe eine Phosphonat- oder Phosphoramiditgruppe ist, wobei die funktionelle Gruppe über ein Sauerstoffatom mit dem Abstandshalter, der mindestens eine Kohlenstoff enthaltende Gruppe umfasst, direkt verbunden ist. Weiterhin stellt die vorliegende Erfindung eine Vorrichtung zur Nukleinsäureanalytik bereit, umfassend einen unteren Teil, der eine flüssige Phase aufnehmen kann und einen oberen Teil, der nicht dauerhaft mit dem unteren Teil verbunden und in den unteren Teil einsetzbar ist, wobei der obere Teil mindestens zwei voneinander abgetrennte Kompartimente aufweist.

Schließlich betrifft die vorliegende Erfindung Verfahren zur Isolierung von Nukleinsäuren aus Proben bzw. Verfahren zum Nachweis von Nukleinsäuren in Proben.

### Kurze Beschreibung der Abbildungen

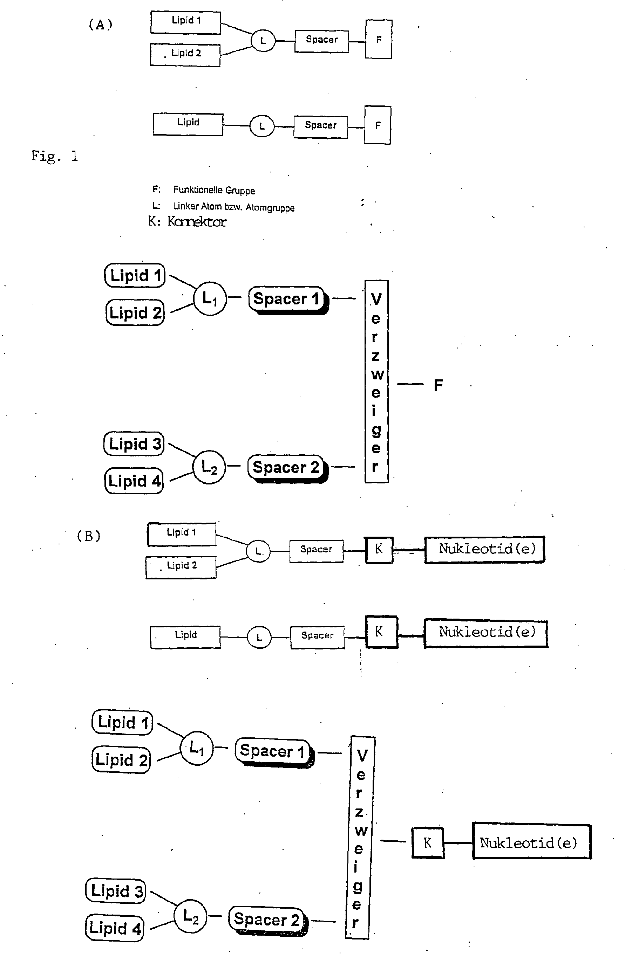

Die Figur 1 zeigt schematisch den Aufbau der erfindungsgemäßen Verbindungen. In
(A) ist die erfindungsgemäße Verbindung, das reaktive Lipid, umfassend einen oder mehrere lipophile Anteile, die funktionelle Gruppe und der dazwischen liegende Linker und Abstandshalter gezeigt. Dargestellt sind verschiedene Ausführungsformen der reaktiven Lipide, bei denen sowohl ein- als auch doppel- oder mehrkettige Lipide in den Verbindungen vorhanden sind.

Die Figur 1 (B) stellt die erfindungsgemäßen Nukleolipide dar. Auch hier sind verschiedene Ausführungsformen der Nukleolipide dargestellt, bei denen sowohl einals auch doppel- oder mehrkettige Lipidanteil(e) in den Verbindungen vorhanden sind.

Die Figur 2 (A) stellt den Syntheseweg eines erfindungsgemäßen reaktiven Lipids, hier eines Phosphoramiditderivats, dar, siehe auch Beispiel 1. (B) stellt erfindungsgemäße verzweigte reaktive Lipide dar (Verbindungen 4 + 5)

Figur 3 stellt schematisch eine erfindungsgemäße Vorrichtung zur DNA-Analyse dar.

Die Abbildung 4 zeigt schematisch die Funktionsweise der erfindungsgemäßen Vorrichtung und des Verfahrens zum Nachweis von spezifischen Sequenzen (TargetNukleinsäuren) in einer Probe.

### Detaillierte Beschreibung der Erfindung

Die vorliegende Erfindung betrifft Nukleolipide sowie Zwischenprodukte zu deren Herstellung. Unter einem Nukleolipid wird eine chemische Verbindung aus einem Nukleosid, einem Nukleotid oder einer Nukleobase und einem ein-, doppel- oder mehrkettigen Lipid verstanden. Das oberflächenaktive Verhalten derartiger "chimärer" Moleküle wurde üblicherweise in Langmuir-Blodgett Apparaturen untersucht. Es hat sich gezeigt, dass derartige Verbindungen sich an der Phasengrenzfläche so orientieren, dass der hydrophobe Molekülteil (Lipid) in die Gasphase weist, die hydrophile Kopfgruppe hingegen in die flüssige Subphase.

Die Synthese von Nukleolipiden und ihr Spreitungsverhalten in der Phasengrenze Flüssigkeit-Gas ist im Stand der Technik beschrieben. In der vorliegenden Anmeldung werden neue Nukleolipide beschrieben, die besonders gut zur Nukleinsäureanalytik geeignet sind. Die erfindungsgemäßen Nukleolipide umfassen einen lipophilen Anteil und eine funktionelle Gruppe, die über einen Linker und einen Abstandshalter miteinander verbunden sind, und ein Nukleosid, ein Oligonukleotid oder Polynukleotid, dadurch gekennzeichnet, dass der Linker eine Amingruppe oder ein Molekül mit mindestens zwei funktionellen Gruppen ist und mit dem lipophilen Anteil verbunden ist, und die funktionelle Gruppe ein Phosphorsäurediester ist, wobei die funktionelle Gruppe über ein Sauerstoffatom mit dem Abstandshalter, der mindestens eine Kohlenstoff enthaltende Gruppe umfasst, verbunden ist.

In einer bevorzugten Ausführungsform der vorliegenden Erfindung beinhalten die erfindungsgemäßen Nukleolipide eine Oligonukleotidstruktur mit 2 bis 100 Nukleotiden.

Weiterhin betrifft die vorliegende Erfindung Verbindungen, reaktive Lipide, die bei Umsetzung mit einem Nukleosid, einem Oligonukleotid oder Polynukleotid ein Nukleolipid ausbilden. Diese reaktiven Lipide umfassen einen lipophilen Anteil und eine funktionelle Gruppe, die über einen Linker und einen Abstandshalter miteinander verbunden sind, dadurch gekennzeichnet, dass der Linker eine Amingruppe oder ein Molekül mit mindestens zwei funktionellen Gruppen ist und mit dem lipophilen Anteil kovalent verbunden ist und die funktionelle Gruppe eine Phosphonat- oder Phosphoramiditgruppe ist, wobei die funktionelle Gruppe über ein Sauerstoffatom mit dem Abstandshalter, der mindestens eine Kohlenstoff enthaltende Gruppe umfasst, kovalent verbunden ist.

Bevorzugt weist diese erfindungsgemäße Verbindung folgende Struktur auf: 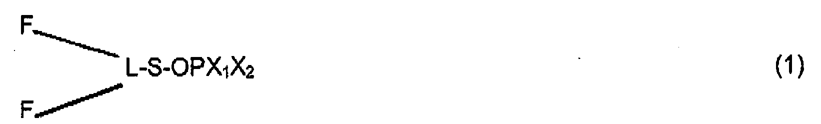
mit F = lipophiler Anteil
L = tertiärem Amin- oder Glycerinrest
S = Abstandshalter, der mindestens eine Kohlenstoff enthaltende Gruppe umfasst; und
OPX₁X₂ = Phosphonat- oder Phosphoramiditgruppe.

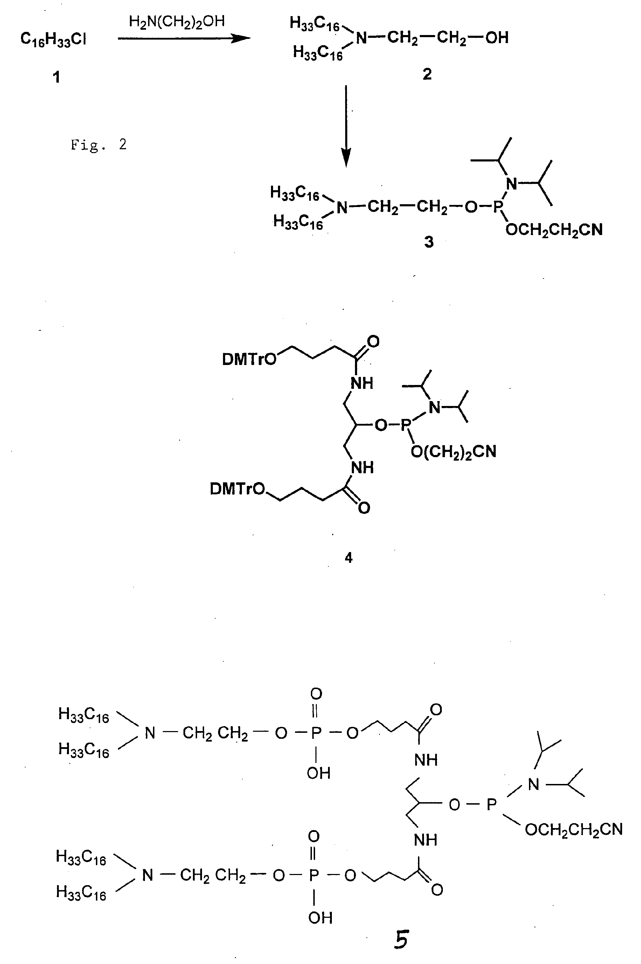

Bevorzugt weist dabei der lipophile Anteil eine Kohlenstoffkette mit 10 bis 40 Kohlenstoffatomen, bevorzugter mit 12 bis 20 Kohlenstoffatomen auf. Die Kohlenstoffkette kann ein oder mehrere konjugierte oder nicht-konjugierte Doppel- oder Dreifachbindungen enthalten, wenn die Verbindung mehrkettige, wie doppelkettige Lipide umfasst, so können die darin enthaltenen lipophilen Anteile gleich oder verschieden sein. Der lipophile Anteil kann aber irgendein Lipid sein, die im Allgemeinen im Wasser unlöslich sind. Hier seien beispielhaft genannt: Kohlenwasserstoffe, wie Squalene und Carotinoide; Alkohol, wie Waschalkohole und Cholesterin; Ether, Carbonsäuren, gesättigte und ungesättigte Fettsäuren; Terpene und Steroide, sowie lipophile Vitamine, wie Vitamin D, E und D2. etc. Die Zahl der Lipidgruppen kann auch durch Verwendung von dendritischen Verzweigungsmolekülen weiter erhöht werden, um die Lipophilie der Verbindungen der Lipid-Nukleinsäure-Konjugate zu erhöhen (siehe z.B. Verbindung 5 in Figur 2).

Die Linkergruppe ist bevorzugt ein tertiäres Amin. Eine weitere bevorzugte Linkergruppe ist ein Glycerin. Weitere beispielhafte Linkergruppen sind ein sekundäres Amin, mehrwertige Alkohole, wie drei- oder vierwertige Alkohole, insbesondere 1,3,5-Trihydroxypentan oder 1,4,7-Trihydroxyheptan oder Pentaerythrit.

Der Abstandshalter umfasst mindestens eine Kohlenstoff enthaltende Gruppe und ist bevorzugt eine Alkyl- oder Alkenylgruppe mit 1 bis 10 Kohlenstoffatomen, insbesondere bevorzugt eine Ethylengruppe. Natürlich kann der Abstandshalter auch andere Gruppen enthalten, solange dieser die Spreitung der mit diesen Verbindungen hergestellten Nukleoüpide an einer Grenzfläche nicht einschränkt.
Die funktionelle Gruppe ist bevorzugt eine Phosphoramiditgruppe.

Eine besonders bevorzugte Ausführungsform der erfindungsgemäßen Verbindung weist ein tertiäres Amin als Linker auf, eine C₁-C₁₀ Alkylgruppe als Abstandshalter, ein Phosphoramidit als funktionelle Gruppe, und die Lipide umfassen eine Kohlenwasserstoffkette mit 12 bis 20 Kohlenstoffatomen, wobei diese Kette(n) ein oder mehrere konjugierte und nicht-konjugierte Doppelbindungen enthalten können und wobei gegebenenfalls die Kohlenstoffketten gleich oder verschieden sein können.

Mit Hilfe dieser erfindungsgemäßen Verbindungen werden nach bekannten Verfahren Nukleolipide hergestellt. Dabei umfassen bevorzugte Ausführungsformen der erfindungsgemäßen Nukleolipide solche, die mit bevorzugten Ausführungsformen der erfindungsgemäßen reaktiven Lipide hergestellt wurden. In einer besonders bevorzugten Ausführungsform ist der lipophile Anteil mit dem Linker und dem Abstandshalter über eine Phosphordiestergruppe mit dem 5'-Ende des Oligo- oder Polynukleotids verbunden.

Die vorliegende Erfindung betrifft weiterhin ein Verfahren zur Herstellung von modifizierten Nukleotiden, Oligonukleotiden oder Polynukleotiden, umfassend den Schritt des Umsetzens der erfindungsgemäßen reaktiven Lipide mit einem außer an einer OH-Gruppe geschützten Nukleosids, Oligonukleotids oder Polynukleotids. Dies erlaubt die Herstellung von Nukleolipiden, insbesondere der erfindungsgemäßen Nukleolipide.

Zur Darstellung der Lipid-Probennukleinsäure-Konjugate werden zunächst in einer dem Fachmann bekannten Weise die verschiedenen Probennukleinsäure-Einzelstränge aufgebaut. Vorzugsweise geschieht dies durch automatische Festphasensynthese unter Anwendung der Phosphoramidit- bzw. der Phosphonattechnik. Ein Lipid wird abschließend bei der routinemäßigen automatischen Synthese als letzter Synthesebaustein mittels eines erfindungsgemäßen Phosphoramiditderivates oder auch mit Hilfe von entsprechenden Phosphonaten angehängt. Wie ausgeführt kann die Lipidkomponente verschiedene Strukturen besitzen, Beispiele hierfür sind in der Figur 1 dargestellt.

Die vorliegende Erfindung betrifft weiterhin ein Verfahren zum Nachweis von Nukleinsäuren. Dieses Verfahren umfasst die Schritte des Bereitstellens einer Probe, die möglicherweise Nukleinsäuren enthält, das Bereitstellen von erfindungsgemäßen Nukleolipiden, die unter hybridisierenden Bedingungen mit der nachzuweisenden Nukleinsäure hybridisiert und in Kontakt bringen der Probe mit den Nukleolipiden unter hybridisierenden Bedingungen, um ein Hybridisierungsprodukt einer Nukleinsäure aus der Probe und dem ertndungsgemäßen Nukleolipiden auszubilden und der Nachweis des Hybridisierungsprodukts. In diesem Zusammenhang bedeutet der Ausdruck "Hybridisierung" oder "hybridisierende Bedingungen" Hybridisierung unter herkömmlichen Hybridisierungsbedingungen, insbesondere unter stringenten Bedingungen, wie sie z.B. Sambrook and Russell (2001), Moleculare cloning: a labratory manual, CSH Press. Cold Spring Harbor, N.Y., USA, beschrieben sind. In einer besonders bevorzugten Ausführungsform bedeutet der Ausdruck "Hybridisierung", dass eine Hybridisierung unter den folgenden Bedingungen erfolgt:

| | |
|---|---|
| Hybridisierungspuffer: | 2 x SSC; 10 x Denhardt-Lösung |
| | (Ficoll 400 + PEG + BSA; Verhältnis 1:1:1); 0,1 % SDS; 5 mMol EDTA; 50 mMol Na₂HPO₄; |
| | 250 µg/ml Heringssperma DNA; 50 µg/ml tRNA; |
| | oder |
| | 0,25 mol Natriumphosphatpuffer, pH 7,2; |
| | 1 mMol EDTA, |
| | 7 % SDS |
| Hybridisierungstemperatur: | T = 60 °C |
| Waschpuffer: | 2 x SSC; 0,1 % SDS; |
| Waschtemperatur: | T = 60 °C |

In einer besonders bevorzugten Ausführungsform hybridisiert die Probensequenz mit der Targetsequenz nur bei Hybridisierungsbedingungen, bei denen die Probensequenz komplementär zu der Targetsequenz ist.

Weiterhin betrifft die vorliegende Erfindung ein Verfahren zum Nachweis des Vorhandenseins oder der Abwesenheit von Nukleinsäuren mit spezifischen Sequenzen in einer Probe umfassend die Schritte: in Kontakt bringen der Probe mit erfindungsgemäßen Nukleolipiden von Oligo- oder Polynukleotiden, wobei mindestens eine der Oligo- oder Polynukleotide eine Sequenz aufweist, die im Wesentlichen komplementär zu einer spezifischen Sequenz der Nukleinsäuren in der Probe ist und Nachweis der Ausbildung von Hybridisierungsprodukten der erfindungsgemäßen Nukleolipide und einer spezifischen Sequenz einer Nukleinsäure aus der Probe (Targetsequenz).

Die nachzuweisende Nukleinsäure mit spezifischer Sequenz (Targetsequenz) kann zuvor auf konventioneller und dem Fachmann bekannter Weise mit einer Reportergruppe markiert worden sein. Dem Fachmann sind übliche Reportergruppen oder Markermoleküle bekannt. Beispielhaft seien hier erwähnt: Fluoreszenzfarbstoffe, radioaktive Markierung, Biotin etc.

In einer bevorzugten Ausführungsform ist die Reportergruppe ein Fluoreszenzfarbstoff, z.B. Fluoreszein, ein Mitglied der Alexa-Farbstofffamilie oder der Cy-Farbstofffamilie.

In einer weiteren Ausführungsform wird das erfindungsgemäße Verfahren zum Nachweis der An- oder Abwesenheit von Nukleinsäuren mit spezifischen Sequenzen in einer Probe so durchgeführt, dass das in Kontakt bringen in abgetrennten Bereichen erfolgt, wobei in den einzelnen Bereichen die Nukleotide eine identische Nukleinsäuresequenz aufweisen, so dass in einem Bereich die vorhandenen Probensequenzen identisch sind und in jedem Bereich eine unterschiedliche Sequenz vorliegt.

Im Zuge der Einstellung des chemischen Gleichgewichts hybridisiert die Targetnukleinsäure an die passende Zielsequenz der Vielzahl der Probensequenzen. Gegebenenfalls kann die Hybridisierungskinetik durch Thermostatisierung der flüssigen Phase optimiert werden. Gegebenenfalls können nicht-gebundene Targetsequenzen durch einen Waschprozess entfernt werden.

Erfindungsgemäß kann der Nachweis der Hybridisierungsprodukte durch Messung der Reportergruppe erfolgen. Bei Verwendung eines Fluoreszenzfarbstoffes als Reportergruppe erfolgt die Messung der Hybridisierungsprodukte durch Messung der emittierten Fluoreszenz dieses Fluoreszenzfarbstoffes, In einer bevorzugten Ausführungsform wird dabei die Messung der Reportergruppe so durchgeführt, dass die Messung nur in dem Bereich der Flüssigkeits-Gas Grenzfläche erfolgt. In einer weiteren Ausführungsform, bei der eine zweite flüssige Phase verwendet wird, die nur gering oder überhaupt nicht mit der ersten flüssigen Phase vermischbar ist und eine Grenzfläche ausbildet, kann die Messung der Reportergruppe an der Flüssigkeit-Flüssigkeit Grenzfläche zwischen den beiden Flüssigkeiten erfolgen.

Die erfindungsgemäßen Nukleolipide eignen sich nicht nur zum Nachweis von Nukleinsäuren in Proben, sondem auch zum Isolieren von Nukleinsäuren aus einer nukleinsäurehaltigen Probe. Somit betrifft die vorliegende Erfindung weiterhin ein Verfahren zum Isolieren von Nukleinsäuren aus einer nukleinsäurehaltigen Probe, umfassend die Schritte des a) in Kontaktbringen der nukleinsäurehaltigen Probe mit Nukleolipid(en) in einer ersten flüssigen Phase, die eine Hybridisierung mindestens eines Teils der Nukleinsäuren in der Probe mit den Nukleolipiden erlaubt; b) Zufügen einer zweiten flüssigen Phase, die in Verbindung mit der ersten flüssigen Phase eine flüssig-flüssig Grenzfläche ausbildet, so dass eine Spreitung an der Phasengrenze erfolgt, dabei ragt der lipophile Anteil in die lipophilere flüssige Phase oder in der Gasphase, während der andere Teil des Nukleolipids, an dem die Nukleinsäuresequenz aus der Probe hybridisiert ist, in die andere Flüssigkeit ragt; c) gegebenenfalls Abtrennen der Hybridisierungsprodukte von den übrigen Bestandteilen der Probe und gegebenenfalls Waschen der Hybridisierungsprodukte.

In einer bevorzugten Ausführungsform des erfindungsgemäßen Verfahrens werden die Nukleolipide zur Isolierung von RNA-Molekülen, insbesondere von mRNA-Molekülen verwendet. Bei der Isolierung von mRNA-Molekülen weist dabei der Nukleotidanteil der Nukleolipide eine polydT-Sequenz auf.

Die Vorliegende Erfindung betrifft weiterhin Kits zum Nachweis von Nukleinsäuren, die ein oder mehrere der erfindungsgemäßen Nukleolipide enthalten. Diese Kits enthalten weiterhin Anweisungen zur Durchführung des Nachweises von Nukleinsäuren und gegebenenfalls eine zweite flüssige Phase, die mit der flüssigen Probe eine Flüssig-Flüssig Grenzfläche ausbildet.

Die erfindungsgemäßen Nukleolipide sind weiterhin zur Herstellung von Nukleinsäurearrays geeignet. D.h., die Nukleolipide mit einem Lipidanteil und einem Oligonukleotidanteil können in Nukleinsäurearrays, sogenannten DNA-Chips, Verwendung findet. Diese DNA-Chips eignen sich zur Analyse von Genen bzw. Genabschnitten. Mit Hilfe der Nukleolipide ist es nun möglich, solche Microarrays herzustellen, die im Gegensatz zu bisher beschriebenen DNA-Chips komplizierte chemische Verfahren zur Aktivierung der festen Oberfläche und zu chemischen Fixierung funktionalisierter Probensequenzen an diesen Oberflächen nicht mehr notwendig machen.

Somit betrifft die vorliegende Erfindung in einem weiteren Aspekt eine Vorrichtung zur Nukleinsäureanalytik, z.B. ein DNA-Microarray, umfassend einen unteren Teil, der eine flüssige Phase aufnehmen kann und einen oberen Teil, der nicht dauerhaft mit dem unteren Teil verbunden ist und in den unteren Teil einsetzbar ist, wobei der obere Teil mindestens zwei voneinander abgetrennte Kompartimente aufweist.

Bevorzugt weist der obere Teil mindestens 4, 8, 16, 25, 64, 256, 384, usw. abgetrennte Kompartimente auf.

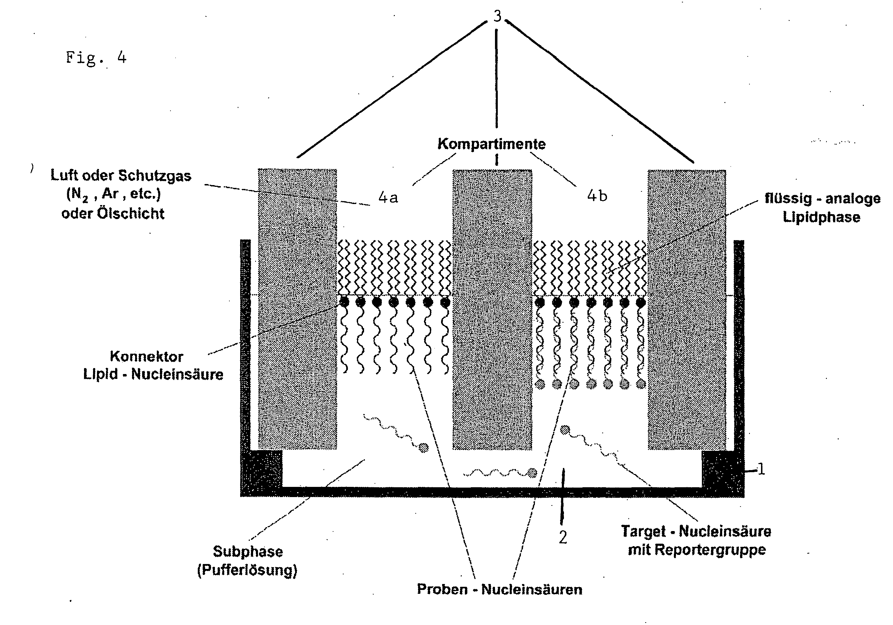

In einer bevorzugten Ausführungsform dieser Vorrichtung ist der obere Teil so ausgebildet, dass, wenn er in dem unteren Teil eingesetzt wird, der untere Abschnitt der im unteren Teil vorliegenden flüssigen Phase nicht in einzelne Kompartimente unterteilt ist. Dies ist z.B. in der Abbildung 4 dargestellt. Abbildung 4 stellt einen Querschnitt einer erfindungsgemäßen Vorrichtung dar. Der untere Teil 1 umfasst einen Bereich, in dem die erste flüssige Phase 2 vorliegt. Der obere Teil 3 unterteilt hier den oberen Bereich der ersten flüssigen Phase in zwei Kompartimente 4a und 4b. Der untere Bereich der ersten flüssigen Phase wird nicht durch den oberen Teil 3 in einzelne Kompartimente unterteilt. Hier können sich die in der ersten flüssigen Phase vorhandenen Targetnukleinsäuren mit der Reportergruppe frei bewegen. Gegebenenfalls ist eine Rührvorrichtung in diesem unteren Bereich angeordnet.

In den Kompartimenten 4a und 4b liegen die Probennukleinsäuren gespreitet an der Grenzfläche der ersten Flüssigkeit mit einem Gas oder mit der zweiten Flüssigkeit vor, wobei die Probennukleinsäuren in die erste Flüssigkeit hineinragen.

Mit Hilfe der erfindungsgemäßen Vorrichtung kann nun ein spezifischer Nachweis der Targetnukleinsäuren durchgeführt werden. Im Folgenden wird beispielhaft der Ablauf einer Nukleinsäureanalytik mit Hilfe der erfindungsgemäßen Vorrichtung beschrieben.

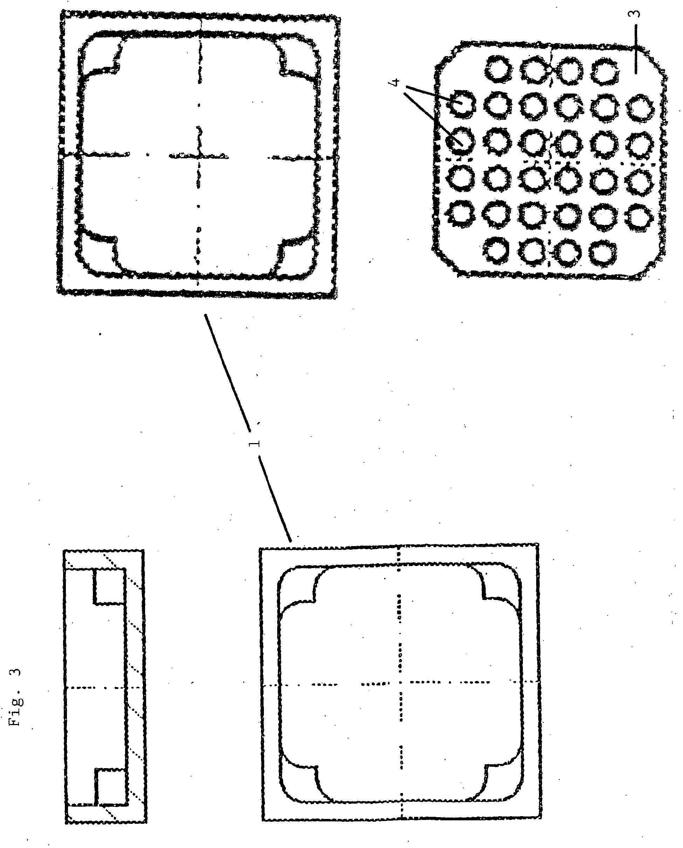

Die erfindungsgemäßen, hoch lipophilen Oligonukleotid-Einzelstränge verschiedener Nukleotidsequenzen werden einzeln in die Kompartimente der in Fig. 3 abgebildeten Apparatur z.B. mit Hilfe eines Spotters eingebracht und bilden dort eine monomolekulare Schicht mit den Eigenschaften einer flüssig-analogen Phase. Die Lipidmoleküle weisen dabei in die Gasphase - die Oligonukleotide in die flüssige Phase (Abb. 4). Gegebenenfalls kann die wässrige Phase mit einer dünnen Ölschicht überschichtet werden, so dass die Lipidketten in die lipophile Phase weisen und sich eine flüssig-flüssig Phasengrenze ausbildet.

Die zu identifizierende Targetsequenz, die zuvor auf konventionelle und dem Fachmann bekannte Weise mit einer Reportergruppe z.B. einem Fluoreszenzfarbstoff versehen wurde, wird anschließend in die allen Probensequenzen gemeinsame Subphase injiziert und dort durch leichtes mechanisches Rühren verteilt. Im Zuge der Einstellung des chemischen Gleichgewichtes hybridisiert die Target-DNA an die passende Zielsequenz. Gegebenenfalls kann die Hybridisierungskinetik durch Thermostatisierung des unteren, die Subphase enthaltenden Teils der Apparatur und/oder durch anschließende Durchströmung der Subphase mit einer Pufferlösung (Waschprozess) optimiert werden. Im entsprechenden Kompartiment der Vorrichtung, in der die Hybridisierung zwischen optimal passender und bekannter Probensequenz und markierter Targetsequenz erfolgt, ist letztere dann mittels dem Fachmann bekannter Detektionsverfahren, z.B. mittels eines Fluoreszenzdetektors, zu identifizieren.

Die in Abb. 3 schematisch abgebildete Vorrichtung zur DNA-Analyse kann auf vielfältige Weise modifiziert werden. Die Möglichkeiten der Modifikation betreffen die Zahl, Form und Maße der Kompartimente für die Aufnahme der Probensequenzen, das Volumen der Subphase sowie das Material der Apparatur. Letzteres kann beispielsweise Plexiglas, Edelstahl oder Quarzglas sein. Zur Thermostatisierung der Subphase kann die Vorrichtung in ein Wärmebad gesetzt werden; die Temperierung kann jedoch auch auf thermoelektrischem Wege (Pelltier-Element) geschehen. Die Temperierung kann also sowohl intern als auch extern erfolgen.

In einer bevorzugten Ausführungsform umfasst die Vorrichtung eine Spottingeinheit zum Einbringen von Nukleolipiden mit spezifischer Nukleinsäuresequenz, insbesondere der Nukleolipide gemäß der vorliegenden Erfindung, in die einzelnen Kompartimente. In einer weiteren Ausführungsform umfasst die erfindungsgemäße Vorrichtung eine Einheit zur Detektion der Reportergruppen, die in den Hybridisierungsprodukten aus Targetingsequenz und Probensequenz vorhanden sind. An diese a) Detektionseinrichtung kann sich b) eine Auswerteanrichtung anschließen. Diese Anordnung erlaubt gegebenenfalls eine Echtzeitanalytik der Hybridisierungseigenschaften und eine halb-quantitative bzw. quantitative Bestimmung der Targetingsequenzen in der zu analysierenden Probe.

Wie bereits oben ausgeführt eignen sich die erfindungsgemäßen reaktiven Lipide insbesondere zum Einsatz in herkömmlichen DNA-Synthesizern, wo sie als 5'-End-Building-Block eingesetzt werden können. Dies erlaubt eine einfache Synthese der Probensequenzen und vermindert die Problematik der Neosynthese der Oligonukleotide auf dem Array bzw. die schwierige chemische Fixierung vorher funktionalisierter Nukleinsäureproben auf aktivierten Oberflächen des Arrays.

Bei der Auswahl der Probennukleinsäuren besteht eine große Variationsbreite.

Hier können nicht nur aus der Natur abgeleitete DNA- und RNA-Moleküle zum Einsatz kommen. Vielmehr können auch Oligomere verwendet werden, die in mannigfaltiger Weise modifiziert sind. So kann beispielsweise zur Hybridisierung mit der zu analysierenden Target-Nukleinsäure eine komplementäre PNA (Peptid-Nukleinsäure) eingesetzt werden. Darüber hinaus wurden in den letzten Jahren verschiedene im Zuckerteil modifizierte Nukleinsäuren, z.B. Hexose- bzw. Hexitol-Nukleinsäuren, dargestellt, die zur Hybridisierung mit natürlichen Nukleinsäuren befähigt sind. Auch solche Proben-Nukleinsäuren können im hier eröffneten Analyseverfahren zum Einsatz kommen. Des weiteren wurde gezeigt, dass durch Modifikation der Nukleobasen eines Proben-Oligonukleotids, so z.B. durch Inkorporation von purin-isosteren 8-Aza-7-desaza-7-halogenopurin-Basen, die Stabilität eines Duplexes mit einem herkömmlichen DNA-Oligomer erheblich erhöht werden, und somit eine Harmonisierung der ansonsten unterschiedlich stabilen Guanin-Cytosin und Adenin-Thymin Basenpaare erreicht werden kann. Auch derartig modifizierte Proben-Nukleinsäuren können im hier eröffneten Verfahren zum Einsatz kommen.

Die Gasphase oder das Gas ist erfindungsgemäß Luft oder ein Schutzgas, wie N₂, Ar, etc. Wenn erfindungsgemäß ein System mit zwei flüssigen Phasen verwendet wird, so sind diese beiden Phasen im Wesentlichen nicht mischbar und bilden eine Grenzschicht, die flüssig-flüssig Grenzfläche, aus. Dabei wird eine lipophile Phase und eine hydrophile Phase erhalten. Üblicherweise befinden sich die Targetsequenzen in der hydrophilen Phase, die üblicherweise eine wässrige Phase, wie eine Pufferlösung ist. Die lipophile Phase wird z.B. durch ein organisches Lösungsmittel oder Öl ausgebildet. Dem Fachmann sind entsprechende Systeme bekannt.

Im Folgenden wird beispielhaft die Synthese einer erfindungsgemäßen Verbindung beschrieben. Es ist klar, dass die vorliegende Erfindung nicht auf die Beispiele beschränkt ist.

### Beispiel

2-Cyanoethyl-2-(dihexadecanylamino)ethyl dlisopropylamidophosphan (3) 1 mmol (632.1 mg) des durch Umsetzung von 1-Chlorohexadecan mit Ethanolamin dargestellten Bis(hexadecanyl)-2-hydroxyethylamin wird in wasserfreiem Dichlormethan (25 ml) gelöst und mit Diisopropylamin (330 µl, 1.95 mmol) und Chloro-(2-cyanoethoxy)(diisopropylamino)phosphin (320 µl, 1.5 mmol) versetzt. Nach 40 minütigem Rühren bei Raumtemperatur fügt man 12 ml einer 5 proz. wässrigen NaHCO₃-Lösung hinzu und extrahiert die Mischung 3 x mit je 30 ml Dichlormethan. Die organische Phase wird getrocknet (Na₂SO₄), filtriert und eingedampft. Mitteldruckchromatographie (Kieselgel, 6 x 10 cm, 0.5 Bar, CH₂Cl₂-Aceton, 99:1) ergibt nach Eindampfen der Hauptfraktion die disastereoisomere Titelverbindung (65 %) als farblosen Feststoff. ³¹P-NMR (CDCl₃): δ 148.5, 149.0 ppm. ¹H-NMR (CDCl₃): ö 0.91 (t, J = 8 Hz, CH₃); 1,29 - 1,26 (m, CH₂); 1,51 (d, J = 4 Hz, CH₃-iPr); 2,14 (t, J = 6 Hz, CH₂); 2,32 (t, J = 7,2 Hz, CH₂-Et); 2.54 (t, J = 5.8 Hz, CH₂-N); 3,54 (m, CH₂); um 4.0 (2 m, CH₂).

## Patentansprüche

1. Verbindungen umfassend einen lipophilen Anteil und eine funktionelle Gruppe, die über einen Linker und einen Abstandshalter miteinander verbunden sind, **dadurch gekennzeichnet, dass** der Linker eine Amingruppe oder ein Molekül mit mindestens zwei funktionellen Gruppen ist und mit dem lipophilen Anteil verbunden ist und die funktionelle Gruppe eine Phosphonat- oder Phosphoramiditgruppe ist, wobei die funktionelle Gruppe über ein Sauerstoffatom mit dem Abstandshalter, der mindestens eine Kohlenstoff enthaltende Gruppe umfasst, verbunden sind.

2. Verbindung gemäß Anspruch 1 mit der Formel: 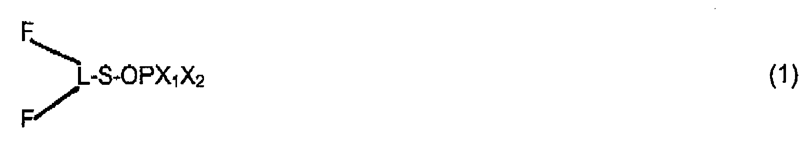
mit F = lipophiler Anteil;
L = tertiärem Amin- oder Glycerinrest;
S = Abstandshalter, der mindestens eine Kohlenstoff enthaltende Gruppe umfasst; und
OPX₁X₂ = Phosphonat- oder Phosphoramiditgruppe.

3. Verbindung gemäß Anspruch 1 oder 2, **dadurch gekennzeichnet, dass** F eine Kohlenstoffkette mit 10 bis 40 Kohlenstoffatomen ist, wobei die Kohlenstoffkette(n) ein oder mehrere konjugierte oder nicht konjugierte Doppel- oder Dreifachbindungen enthalten und wobei die Kohlenstoffketten gleich oder verschieden sein können.

4. Verbindung gemäß einem der vorherigen Ansprüche, wobei der Linker L ein tertiäres Amin ist.

5. Verbindung gemäß einem der vorherigen Ansprüche, wobei die funktionelle Gruppe OPX₁X₂ eine Phosphoramiditgruppe ist.

6. Verbindung gemäß einem der vorherigen Ansprüche, wobei der Abstandshalter S ausgewählt ist aus C₁-C₁₀ Alkyl oder C₁-C₁₀ Alkenyl.

7. Verbindung gemäß einem der vorherigen Ansprüche, wobei
L ein tertiäres Amin ist;
S ist eine C₁-C₁₀ Alkylgruppe;
OPX₁X₂ ist ein Phosphoramidit; und
F ist eine Kohlenwasserstoffkette mit 12 bis 20 Kohlenstoffatomen, wobei diese Kette(n) ein oder mehrere konjugierte oder nicht-konjugierte Doppelbindungen enthalten können und wobei gegebenenfalls die Kohlenstoffketten gleich oder verschieden sein können.

8. Verbindung umfassend einen lipophilen Anteil und eine funktionelle Gruppe, die über einen Linker und einen Abstandshalter miteinander vorbunden sind, und ein Nukleosid, ein Oligonukleotid oder Polynukleotid, **dadurch gekennzeichnet, dass** der Linker eine Amingruppe oder ein Molekül mit mindestens zwei funktionellen Gruppen ist und mit dem lipophilen Anteil verbunden ist, und die funktionelle Gruppe ein Phosphosäurediester ist, wobei die funktionelle Gruppe über ein Sauerstoffatom mit dem Abstandshalter, der mindestens eine Kohlenstoff enthaltende Gruppe umfasst, verbunden ist.

9. Verbindung gemäß Anspruch 8, wobei die Verbindung ein Oligonukleotid mit 2 bis 100 Nukleotiden umfasst.

10. Verbindung gemäß Anspruch 8 oder 9, wobei die funktionelle Gruppe am 5'-Ende des Nukleosids, des Oligonukleotids bzw. des Polynukleotids gebunden ist.

11. Verfahren zur Herstellung von modifizierten Nukleotiden, Oligonukleotiden oder Polynukleotiden, umfassend den Schritt des Umsetzens der Verbindungen gemäß einem der Ansprüche 1 bis 7 mit einem außer an einer OH-Gruppe geschützten Nukleosids, Oligonukleotids oder Polynukleotids unter Erhalt einer Verbindung gemäß einem der Ansprüche 8 bis 10.

12. Verfahren zum Nachweis von Nukleinsäuren umfassend die Schritte:
a) Bereitstellen einer Probe, die Nukleinsäuren enthält,
b) Bereitstellen von Oligonukleotiden oder Polynukleotiden gemäß einem der Ansprüche 8 bis 10, die unter hybridisierenden Bedingungen mit der nachzuweisenden Nukleinsäure hybridisiert;
c) in Kontakt bringen der Probe gemäß a) mit dem Oligo- oder Polynukleotiden gemäß b) unter hybridisierenden Bedingungen, um ein Hybridisierungsprodukt aus einer Nukleinsäure aus der Probe und dem Oligo- oder Polynukleotid gemäß b) auszubilden;
d) Nachweis des Hybridisierungsprodukts aus c).

13. Verfahren zum Nachweis des Vorhandenseins oder der Abwesenheit von Nukleinsäuren mit spezifischen Sequenzen in einer Probe umfassend die Schritte:
a) in Kontakt bringen der Probe mit Oligo- oder Polynukleotiden gemäß einem der Ansprüche 8 bis 10, wobei mindestens eines der Oligo- oder Polynukleotide eine Sequenz enthält, die im Wesentlichen komplementär zu einer spezifischen Sequenz der Nukleinsäuren in der Probe ist;
b) Nachweis der Bildung von Hybridisierungsprodukten aus Oligo- oder Polynukleotiden gemäß einem der Ansprüche 8 bis 10 und einer spezifischen Sequenz einer Nukleinsäure aus der Probe.

14. Verfahren gemäß einem der Ansprüche 12 oder 13, **dadurch gekennzeichnet, dass** die Nukleinsäuren mit spezifischen Sequenzen eine Reportergruppe zum Nachweis der Hybridisierungsprodukte umfasst.

15. Verfahren gemäß Anspruch 14, wobei es sich bei der Reportergruppe um einen Fluoreszenzfarbstoff handelt.

16. Verfahren gemäß einem der Ansprüche 12 bis 15, wobei der Schritt des in Kontakt bringen der spezifischen Nukleinsäuren aus der Probe mit den Verbindungen gemäß Anspruch 8 bis 10 in abgetrennten Bereichen erfolgt, wobei in den einzelnen Bereichen nur Verbindungen gemäß einem der Ansprüche 8 bis 10 mit identischer Nukleinsäuresequenz vorliegen, und jeder Bereich eine Verbindung gemäß einem der Ansprüche 8 bis 10 mit einer unterschiedlichen Nukleinsäuresequenz enthält.

17. Verfahren gemäß einem der Ansprüche 12 bis 16 weiterhin umfassend den Schritt des Waschens der Hybridisierungsprodukte, um nicht gebundene Probensequenzen zu entfemen.

18. Verfahren gemäß einem der Ansprüche 12 bis 17, **dadurch gekennzeichnet, dass** der Nachweis der Hybridisierungsprodukte durch Messung der Reportergruppe, bevorzugt an der Flüssigkeit-Gas Grenzfläche, erfolgt.

19. Verfahren gemäß einem der Ansprüche 12 bis 17, **dadurch gekennzeichnet, dass** der Nachweis der Hybridisierungsprodukte durch Messung der Reportergruppe an einer Flüssigkeit-Flüssigkeit Grenzfläche zwischen einer wässrig-hydrophilen und einer lipophilen Flüssigkeit erfolgt.

20. Verfahren zum Isolieren von Nukleinsäuren aus einer nukleinsäurehaltigen Probe, umfassend die Schritte des
a) in Kontakt bringen der nukleinsäurehaltigen Probe mit (einer) Verbindung(en) gemäß einem der Ansprüche 8 bis 10 in einer ersten flüssigen Phase, die eine Hybridisierung mindestens eines Teils der Nukleinsäuren in der Probe mit der/die Verbindungen gemäß einem der Ansprüche 8 bis 10 erlaubt;
b) Zufügen einer zweiten flüssigen Phase, die in Verbindung mit der ersten flüssigen Phase eine flüssig-flüssig-Grenzfläche ausbildet, so dass der lipophile Anteil der Verbindung gemäß einem der Ansprüche 8 bis 10 in eine der beiden Flüssigkeiten ist, während sich der andere Teil der Verbindung, an dem die Nukleinsäuresequenz aus der Probe hybridisiert ist, in der anderen Flüssigkeit vorliegt,
c) gegebenenfalls Abtrennen der Hybridisierungsprodukte von den übrigen Bestandteilen der Probe und gegebenenfalls Waschen der Hybridisierungsprodukte.

21. Verfahren gemäß Anspruch 20, wobei es zur Isolierung von RNA-Molekülen, insbesondere mRNA-Molekülen geeignet ist.

22. Kit zum Nachweis von Nukleinsäuren umfassend eine oder mehrere der Verbindungen gemäß einem der Ansprüche 8 bis 10.

23. Verwendung der Verbindungen gemäß einem der Ansprüche 8 bis 10 zur Herstellung von Nukleinsäurearrays.

24. Vorrichtung zur Nukleinsäureanalytik umfassend einen unteren Teil, der eine flüssige Phase aufnehmen kann, und einem oberen Teil, der nicht dauerhaft mit dem unteren Teil verbunden ist und in den unteren Teil einsetzbar ist, wobei der obere Teil mindestens zwei voneinander abgetrennte Kompartimente aufweist und die Vorrichtung gegebenenfalls eine Temperiervorrichtung für die flüssige Phase besitzt.

25. Vorrichtung gemäß Anspruch 24, wobei der obere Teil so ausgebildet ist, das er, wenn er in den unteren Teil eingesetzt ist, den unteren Abschnitt einer flüssigen Phase, die im unteren Teil vorhanden ist, nicht in einzelne Kompartimente unterteilt.

26. Vorrichtung gemäß einem der Ansprüche 24 oder 25, weiterhin umfassend eine Spotting-Einheit zum Einbringen von Nukleolipiden mit spezifischer Nukleinsäuresequenz, insbesondere solchen gemäß einem der Ansprüche 8 bis 10, in die einzelnen Kompartimente.

27. Vorrichtung gemäß einem der Ansprüche 24 bis 26, die weiterhin eine Einheit zur Detektion von Reportergruppen in Hybridisierungsprodukten aufweist.

28. Vorrichtung gemäß einem der Ansprüche 24 bis 27 weiterhin umfassend eine Auswerteeinrichtung.
